## Europäisches Patentamt
## European Patent Office
## Office européen des brevets

(19)

(11) Publication number: **0 003 495**
**B1**

(12) **EUROPEAN PATENT SPECIFICATION**

(45) Date of publication of patent specification: **23.03.83**

(51) Int. Cl.³: **A 61 M 1/03,** B 01 D 13/00

(21) Application number: **78850025.4**

(22) Date of filing: **13.12.78**

(54) A device for the diffusion of substances between two fluids separated by a semipermeable membrane.

(30) Priority: **02.02.78 SE 7801231**

(43) Date of publication of application:
**22.08.79 Bulletin 79/17**

(45) Publication of the grant of the patent:
**23.03.83 Bulletin 83/12**

(84) Designated Contracting States:
**BE CH DE FR GB IT LU NL SE**

(56) References cited:
DE - B - 1 257 741
FR - A - 1 312 112
FR - A - 2 173 354
FR - A - 2 181 989
GB - A - 1 447 174
US - A - 2 399 471
US - A - 3 326 380
US - A - 3 332 746

(73) Proprietor: **GAMBRO AB**
**Box 10101**
**S-220 10 Lund (SE)**

(72) Inventor: **Stenberg, Kaj Ove**
**Fugavägen 53**
**S-245 00 Staffanstorp (SE)**
Inventor: **Traven, Lars Johan Christian**
**Röde Orms Gränd 3**
**S-223 77 Lund (SE)**

(74) Representative: **Boberg, Nils Gunnar Erik**
**HOLGER CRAFOORD AB Patent Department Box 10101**
**S-220 10 Lund (SE)**

## A device for the diffusion of substances between two fluids separated by a semipermeable membrane

The present invention relates to a device for the diffusion of substances between two fluids separated by semipermeable membranes which membranes are arranged in pairs for conducting a first fluid including oxygen between the membranes of the pairs and a second fluid such as blood outside the membranes with diffusion of one or more substances from the first fluid to the second fluid through the membranes, including spacing elements in the form of plates, separating the pairs of membranes from each other, and intended to work as a so called oxygenator.

The device is characterized in that means are arranged for combining said diffusion with a subsequent direct mixing of the first fluid into the second fluid and in that said spacing plates are provided with channels which are adapted so as to conduct the second fluid mixed with bubbles of said first fluid along the outside of the membranes in the said pair of membranes.

The construction according to the present invention differs from the prior art as described in e.g. US patent 3 332 746, in that the treated liquid such as blood is fed outside the membranes in each pair of membranes in spacing plates provided with such channels that they can conduct the treated fluid mixed with bubbles of the first fluid for the treatment.

The same differences are true as regards the prior art as e.g. the dialyzer described in US patent 3 326 380.

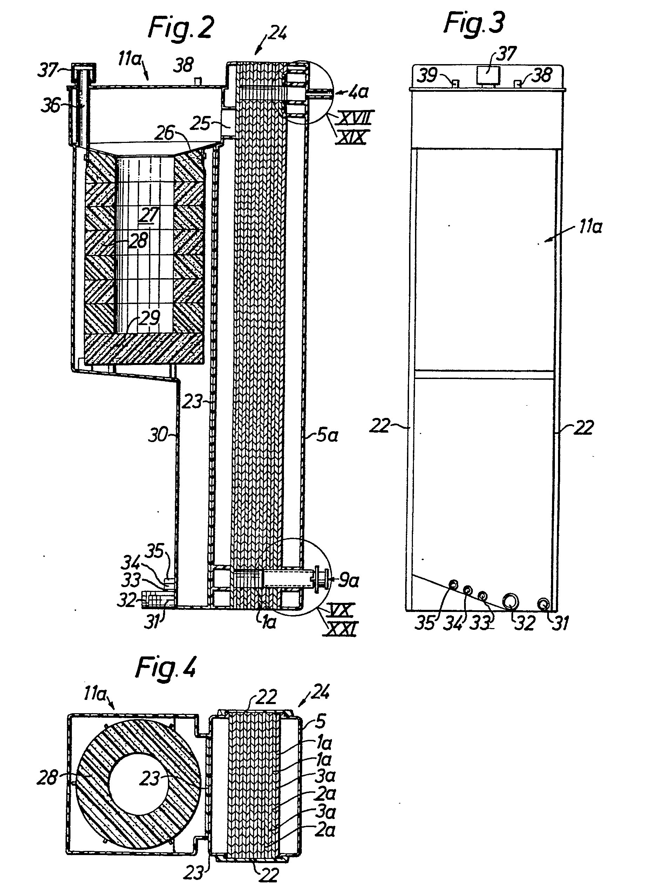

The French patent 1 312 112 on the other hand describes in its fig. 3, for instance, an oxygenator including diffusion of oxygen combined with the direct mixing of blood and oxygen. The capacity of this construction has, however, to be very limited due to its technical construction. The construction according to the present invention on the other hand has a very high capacity due to the fact that as well the diffusion as the direct mixing can be spread out over very large areas in thin layers.

The term fluid, in the foregoing as well as in the following, relates to gases as well as to liquids. In the first place, however, the device in accordance with the invention is intended to work as a so-called oxygenator, the said first fluid consisting of air, oxygen or some other oxygen mixture, whilst the second fluid consists of blood, which is to be oxygenated. It will be clear, however, to those versed in the art, that the device can also be used in other cases where a successive mixing of a first fluid into a second is desired, whether it is a matter of a gas into a liquid, a liquid into another liquid or a gas into another gas.

Where oxygenators in particular are concerned it is possible thanks to the invention to produce in the first place a very fine distribution on microscopic bubbles of the gas mixture used in the blood, and to introduce subsequently the gas mixture in the form of larger bubbles. Owing to the microscopic bubbles an effective oxygenation of the blood is achieved, which is further improved by the large bubbles which in addition facilitate the elimination of carbon dioxide from the blood.

By way of information it may be mentioned in this connection, that in order to avoid complete elimination of carbon dioxide a small amount of carbon dioxide, e.g. 2 to 5%, is usually added to the gas mixture used, which otherwise consists of oxygen gas.

As in dialyzers of the parallel plate-type, spacing elements in the form of plates provided with channels or the like may be arranged in the device in accordance with the invention between the pair of membranes. These plates may be adapted so that they conduct the second fluid, that is to say, the blood, along the outside of the membranes in the said pair of membranes and preferably in counter-flow in relation to the first fluid which is conducted between the membranes in the respective pair of membranes. In this respect the construction in accordance with the invention thus differs from dialyzers which usually conduct the blood enclosed between two membranes or in tubes or fibres of membrane material.

Preferably at least one membrane in each pair of membranes is provided with apertures at its end downstream in respect of the first fluid, for the direct mixing in of the first fluid into the second, that is to say, for the mixing of the gas mixture into the blood. However, this mixing may also take place in a different manner, e.g. through direct injection of the gas mixture into the blood channels, already before the blood comes up to the membranes.

The plates provided with channels are appropriately separated from a third fluid at their side remote from the said pair or membranes by means of further membranes which preferably are also arranged in pairs so as to enclose between them this third fluid. The third fluid may consist, for example, of a gas or a liquid which is intended for the heating or other tempering of the second fluid, that is to say, the blood.

This third fluid, too, is suitably arranged so that it is conducted in counter-flow in respect of the second fluid, that is to say, in respect of the blood.

In a preferred embodiment of the device in accordance with the invention the same comprises elongated membranes arranged in pairs for the said first and third fluids, that is to say, for the gas mixture and for the heating medium respectively, together with plates provided with channels or the like arranged in between for the said second fluid, that is to say, for the blood, the device as a whole being intended to be used in a vertical position. This preferred embodiment is characterized in that the said first and third fluids are adapted to be introduced between the membranes at their upper end, whilst the second fluid, that is to say, the blood, is adapted to be introduced at the lower end of the plates provided with channels.

For the purpose of achieving an effective oxygenation of the blood, an excess of oxygen is supplied. Means are therefore provided to eliminate this excess, before the blood is returned to the patient.

If the device in accordance with the invention comprises two or more plates connected in parallel for the conducting of the said second fluid, that is to say, for the conducting of the blood, it is suitably also provided with means adapted so as to conduct this fluid only to certain ones of the said plates in order to vary the capacity of the device. In this manner one and the same device can be used, for example, for grown persons as well as for children. Such an adjustment can be achieved by providing the plates with aligned apertures which form a shaft passing through a stack of plates for the introduction of the second fluid, channels being arranged in the plates for the conducting of the second fluid from the shaft to the truly active part of the plate. These channels can afterwards be closed with the help of special shut-off elements. Such shut-off elements may consist for example of a tubular plunger penetrating into the shaft to a greater or lesser extent which with its outer side can shut off one or several of the channels.

The invention is described in detail in the following with reference to the enclosed drawings which by way of example describe two different embodiments of the same.

Fig. 1 shows relatively schematically an exploded view of a first embodiment of the subject of the invention.

Fig. 2, 3 and 4 show, also schematically, three views at right angles to one another, partially in section, of a second preferred embodiment of the subject of the invention.

Fig. 5 shows the device in accordance with fig. 2 seen from the right.

Fig. 6 shows part of the same device seen in the direction against the plane of drawing according to fig. 2.

Fig. 7 shows a section along the line VII—VII in fig. 6.

Fig. 8 and 9 show two plane views of a plate, a great number of which enters into the part shown in fig. 6.

Fig. 10 shows a section along the line X—X in fig. 9.

Fig. 11 shows a section along the line XI—XI in fig. 9.

Fig. 12 shows a section along the line XII—XII in fig. 8.

Fig. 13 shows a section along the line XIII—XIII in fig. 9.

Fig. 14 shows a section along the line XIV—XIV in fig. 5.

Fig. 15 shows a section along the line XV—XV in fig. 5.

Fig. 16 shows a section along the line XVI—XVI in fig. 5.

Fig. 17 shows two membranes, a number of which is intended to be arranged in pairs between plates of the type shown in fig. 8 to 13.

Fig. 18 and 19 show on a larger scale a detail from fig. 2 which is to be found within the circle designated XVIII—XIX.

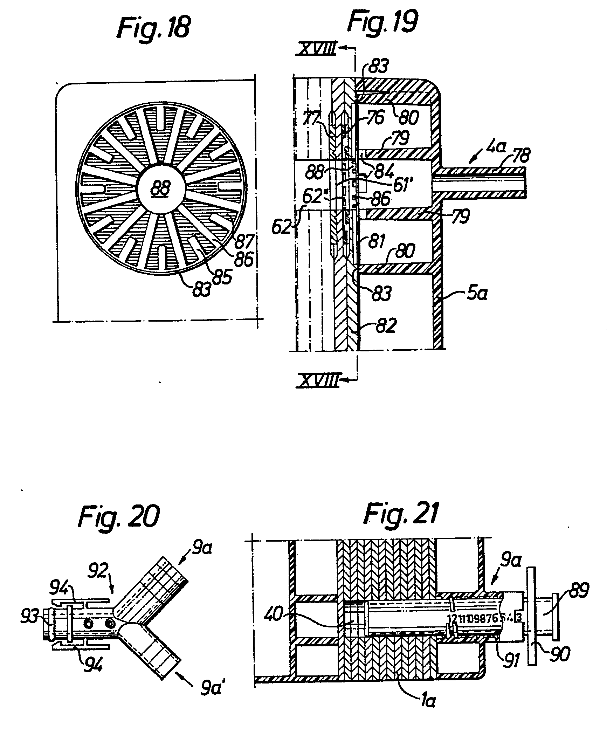

In the same manner in fig. 20 and 21 a detail from fig. 2 is shown which is to be found in the circle designated by numerals XX and XXI.

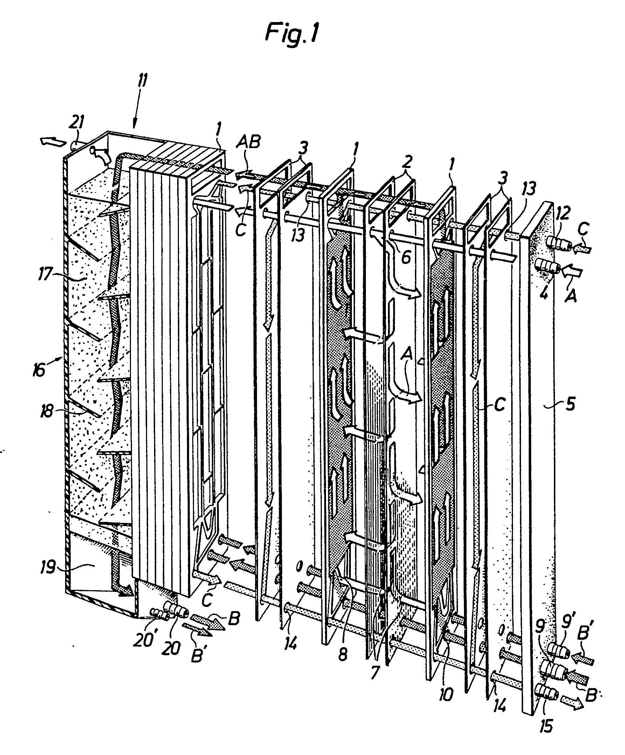

The construction shown in fig. 1 is intended mainly for the oxygenating and tempering of blood. In the following description the different fluids will therefore be referred to as blood, water and gas respectively. As mentioned previously, however, it will be obvious to those versed in the art, that other fluids may also occur. For example, the heating may be replaced by a dialysis if the membranes confining the water are made semipermeable and the water is replaced by dialysis liquid.

As can be seen from fig. 1, the device in accordance with the invention can be made up of a number of plates 1, provided with channels with pairs of membranes, 2 and 3, respectively, arranged between them. The membranes 2 are adapted so as to allow the gas to pass through in the form of microscopic bubbles. The path of the gas is indicated by the arrows marked A. Accordingly, it is supplied via a connecting nozzle 4 in an end plate 5 and is passed to the membranes 2 to be subsequently introduced between them downwards into the device. Thus the gas is introduced between the membranes through apertures 6 and conducted out of the membranes through apertures 7. With the help of channels 8 in the plates 1 the gases are then conducted to be mixed directly into the blood.

The path of the blood has been indicated by arrows marked B. Accordingly, it is introduced via a connecting nozzle 9 into the end plate 5 and is conducted to apertures 10 in the plates 1. From these apertures 10 the blood is conducted further to the truly active part of the plates via channels in a manner described more fully in connection with the embodiment of the subject of the invention described in the following. In the plates 1 the blood is mixed with the gas and it is then discharged in the direction indicated by the arrow AB to a skimming section which is designated as a whole by numeral 11. The arrow B' and the connecting nozzle 9' indicate an extra blood intake which can be used in connection with special operations.

The arrows C show how the water is supplied to and withdrawn from the device. This water is tempered either for cooling or for heating of the blood. Accordingly, the water is introduced via a connecting nozzle 12 into the end plate 5. It is introduced via apertures 13 into the space between the membranes 2 to be then conducted down between these membranes and out via apertures 14 and a connecting nozzle 15 in the end plate 5.

The skimming section 11 in the example shown consists substantially of a vertical shaft 16 which is filled with polyurethane foam 17 with open pores between inclined plates 18. At the bottom the shaft 16 is connected to a reservoir 19 with two outlets 20 and 20' corresponding to the inlets 9 and 9' respectively. Finally, numeral 21 designates an outlet for the skimmed off gas.

In fig. 2—21 a further embodiment of the subject of the invention is shown in more detail. For the parts corresponding to those of the construction in accordance with fig. 1 the same reference designations have been used, but with the addition of an *a*. As can best be seen from fig. 2—4, the construction shown as a further example consists of a number of plates 1a with pairs of membranes, 2a and 3a respectively arranged between them. The plate any membrane packet is held together by a clamping plate or an end plate 5a which with the help of clamping bars 22 is maintained pressed against a skimming unit 11a, the one end wall 23 of which is given substantially the same shape as the clamping plate 5a. Blood is introduced via an inlet 9a, which is shown in greater detail in fig. 20 and 21. In the same manner gas is introduced through an inlet 4a which is shown in greater detail in fig. 18 and 19.

The actual oxygenating part or heating part as a whole has been designated by numeral 24. The oxygenated blood leaves this section via an outlet 25 and flows via a funnel 26 down into a vertical shaft 27 which is formed by rings 28 and a base 29 of polyurethane foam or similar material with open pores. The skimmed off blood then drains into a collecting reservoir 30 and can be discharged via five different outlets 31, 32, 33, 34 and 35. The outlets 33, 34 and 35 are intended for connection to gauges for the measurement of pressure and temperature and for the taking of samples respectively. The actual main outlet is thus the one marked 32. Numeral 31 designates a special outlet which is intended for use in connection with e.g. coronary perfusion. Numeral 36 designates an outlet for skimmed off gas. This outlet is covered by a cap 37. Finally, 38 and 39 designate two inlets which may be utilized for the supply of different media, e.g. heparin, etc.

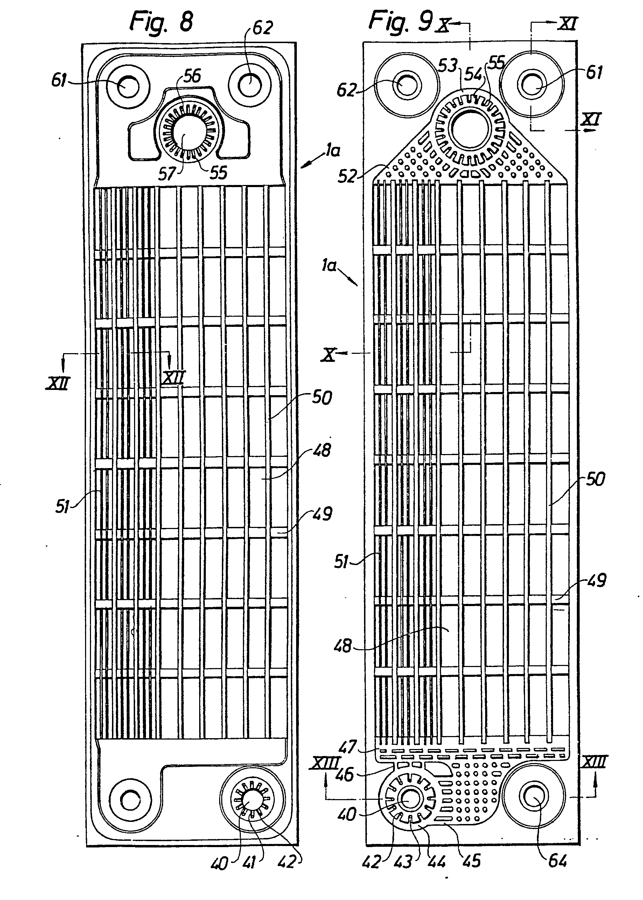

The construction of the plates 1a can best be seen from fig. 8—13. The blood inlet is designated here by numeral 40. From this inlet the blood flows first through the channels designated 41 on the one side (fig. 8) of the plate to continue subsequently through the plate through the smaller holes 42 and then further through the ducts 44, 45, 46 and 47 on the other side (fig. 9) of the plate to the truly active part of the plate which is designated 48.

This active part of the plate 1a comprises transverse bars 49 which uphold longitudinal compression strips 50. Between the compression strips 50 the bars 49 uphold supporting strips 51 which alternately face the two sides of the plate, but which are a little retracted in relation to the outer surfaces of the compression strips 50. The oxygenated blood is subsequently removed via channels 52, 53 and 54 (fig. 9) to the smaller holes 55 which extend through the plate and further on the other side (fig. 8) of the plate through channels 56 to an outlet opening 57.

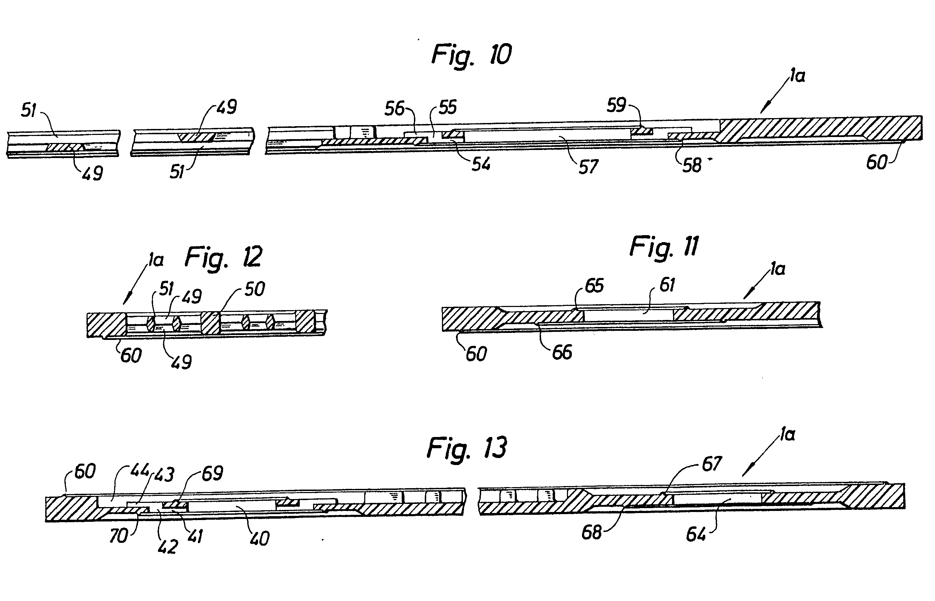

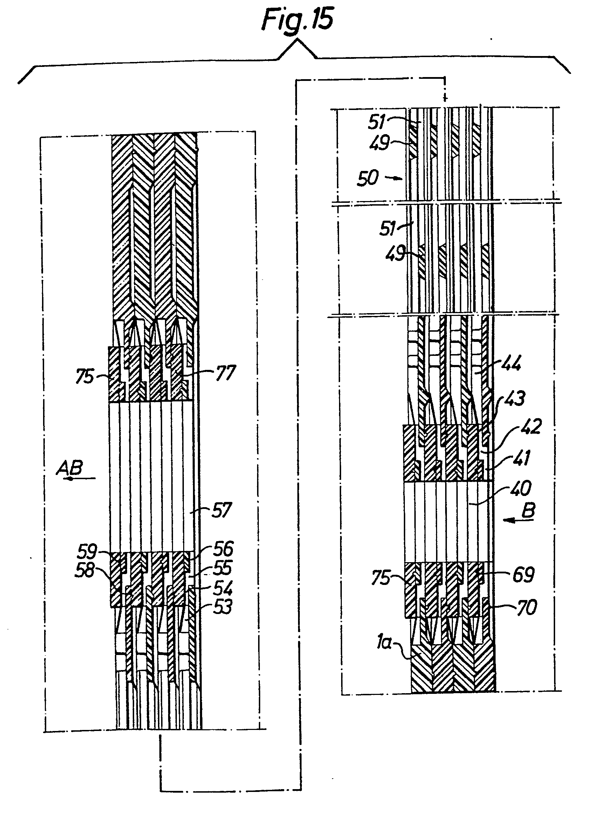

Numerals 58, 59 and 60 designate different sealing beads which are shown most clearly in fig. 10. With the help of these the adjacent membranes are pressed against special packings, which will be described in more detail in connection with fig. 15, or rather against the adjacent plate.

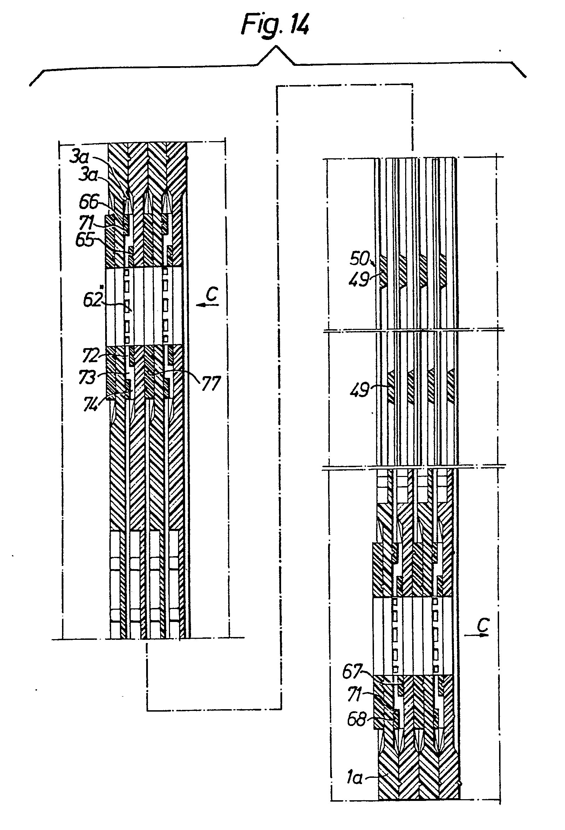

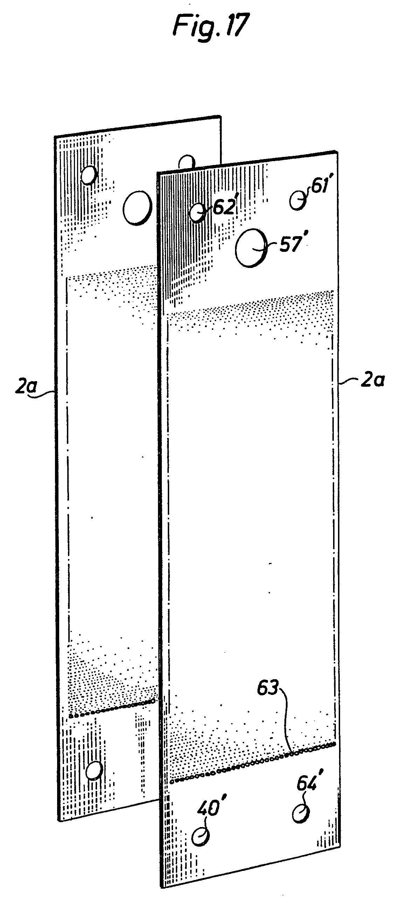

The gas and the water are introduced via openings 61 and 62 respectively and from there further between the membranes with the help of special "buttons" which will be described in more detail in connection with fig. 14—16. The gas is then introduced into the blood via apertures 63 in the membranes 2a, which are shown most clearly in fig. 17. These membranes are provided moreover with further apertures 61', 62', 57', 40' and 64' corresponding to the holes 61, 62, 57, 40 and 64 in the plate 1a. It is appropriate here to compare fig. 17 with fig. 9. Accordingly, numeral 64 designates the outlet for the water. Special buttons are provided here, too, between the membranes which will be described in more detail in connection with fig. 14—16.

Numerals 65 and 66 in fig. 11 and 67 and 68 in fig. 13 designate sealing beads intended to co-operate with the aforementioned "buttons". In the same manner, the sealing beads 69 and 70 shown in fig. 13 are intended to co-operate with special packings.

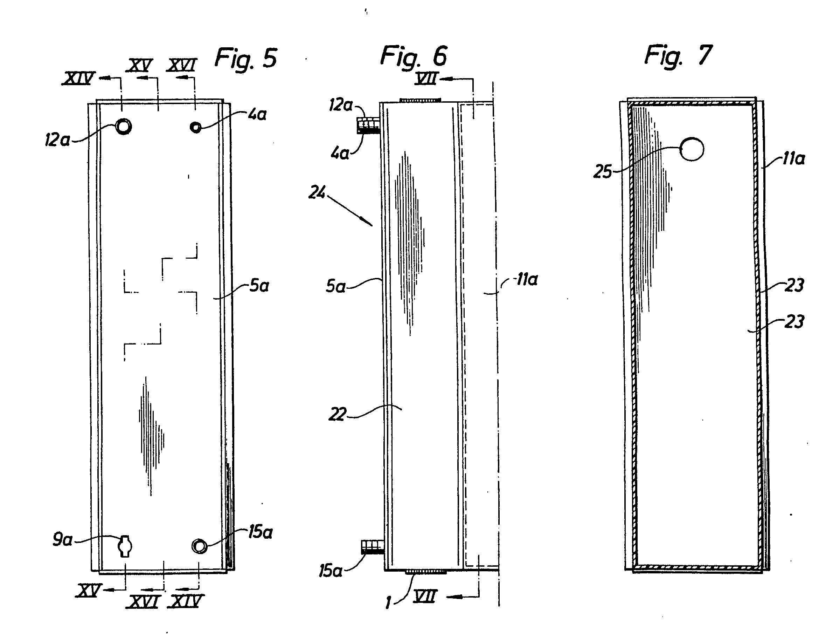

In fig. 5 is shown the device in accordance with fig. 2 seen from the right. Numeral 4a designates the inlet for the gas mixture and 12a the water intake. In the same manner 9a designates the blood inlet and 15a the water discharge. For the rest fig. 5 illustrates essentially only the clamping plate 5a. In addition, however, the position of the sections XIV—XIV, XV—XV and XVI—XVI is shown, that is to say, the sections which are shown in more detail in fig. 14—16.

In fig. 6 the oxygenating section proper of the device is shown which as a whole is designated 24. It is attached to the skimming section 11a with the help of the clamping plate 5a and clamping bars 22.

Fig. 7 shows a section along the line VII—VII in fig. 9. Numeral 25 designates the outlet for the oxygenated blood. The skimming section 11a is situated partly concealed behind its end wall 23 which thus has the same function as the clamping plate 5a.

In fig. 14 is shown how the water is introduced between the membranes 3a, 3a and how it is removed from these membranes with the help of special sealing buttons 71. These buttons, which are placed between the membranes, have a central inner opening 62″ from which the water is conducted through channels 72 on the one side through holes 73 and through channels 74 on the other side. The buttons are adapted so as to seal against sealing beads which are identical with the sealing beads 65 and 66 shown in fig. 11, but which surround the aperture 62. At the outlet end corresponding sealing buttons are intended to be pressed instead against the sealing beads 67 and 68 shown in fig. 13 to form a seal.

In fig. 15 is shown how blood is introduced at the place indicated by the arrow B and discharged in oxygenated state at the place indicated by the arrow AB. The apertures and channels 40, 41, 42, 43 and 44 at the inlet end and 53, 54, 55, 56 and 57 at the outlet end described in fig. 8 and fig. 9 accordingly will be found here. With the help of the sealing beads 69 and 70 at the inlet and 58 and 59 at the outlet the blood flow is effectively sealed off against the other flows with the help of packings 75.

Numeral 77 designates "blind buttons" which are intended to be introduced into the membrane layer where a particular fluid is undesirable. In this manner the gas mixture can be conducted through the pair of membranes 3a, 3a intended for the water without being mixed with the latter and in the same manner the water can be conducted through the pair of membranes 2a, 2a intended for gas without being mixed with the same.

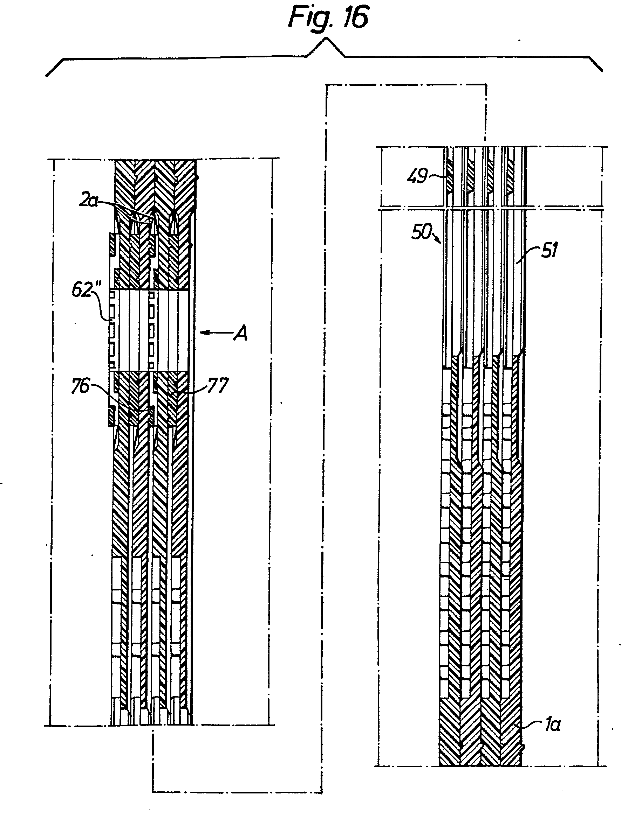

In fig. 16 the inlet for gas is shown. The same is introduced at the place indicated by arrow A and is introduced between the membranes 2a with the help of special buttons 76. These are substantially identical with the buttons 72, and are not therefore described in any detail. The gas mixture is conducted from the membrane 2a and into the blood flow partially by direct diffusion through the membrane partly through a series of holes 63, shown most clearly in fig. 17. This series of very small holes with a diameter of the order of magnitude of 50 $\mu$ produces a high pressure drop and consequently an even distribution of the gas mixture. To bring about the abovementioned diffusion it is preferable to use membranes which are microporous with holes of the order of magnitude of 0.02 $\mu$ and of a thickness of approx. 20—25 $\mu$. Examples of such membranes are various stretched PE or PP membranes or expanded Teflon® membranes. Alternatively, various silicone membranes may be used which are not microporous in the true meaning of the word but wherein the diffusion takes place through the membrane material itself.

In fig. 14—16 are shown again the transverse bars 49, the longitudinal compression strips 50 and the supporting strips 51. The truly active surface of the spacer plates 1a is not shown.

In fig. 18 and 19 the gas inlet 4a is shown in greater detail. The gas mixture used is introduced here via a gas nozzle 78 which is moulded onto the end plate or clamping plate 5a. With the help of two inner concentric nozzles 79 and 80 a filter 81 is pressed against an outer end plate 82, a seal being achieved with the help of a sealing bead 83. With the help of channels 84 the gas mixture is guided over the whole surface of the filter 81. After filtration of the gas is conducted through the channel 62 formed by apertures 61′ and 62″. Examples of usable filters are sintered PE or PP materials of a thickness of approx. 20 $\mu$ and a pre size of appropriately 0.2—0.5 $\mu$. Alternatively, a glass fibre material can be used.

Fig. 18 shows a section along the line XVIII—XVIII in fig. 19 and thus shows the supporting surface proper for the filter 81. This supporting surface, which is thus located inside the sealing bead 83, is formed by radially arranged supporting strips 85 and 86 with channels in between which open out into a central opening 88.

In fig. 20 and 21 is shown schematically how the blood inlet 9a can be adapted for the setting of different capacities. The inlet shown consists of a tubular plunger 89 which with the help of a handwheel 90 and a thread 91 can be adjusted to different depths in the shaft 40 formed by the apertures in the plates. To the plunger 89 is connected a Y-coupling with two inlets 9a and 9a′ corresponding to the inlets 9 and 9′ in the construction according to fig. 1. The seal between the Y-coupling 92 and the plunger 89 is achieved with the help of an O-ring 93 and the actual retention is achieved with the help of hooks 94. In the example shown the device is set for treatment in three spacer plates 1a. The capacity can be increased fourfold, however, by including 12 plates.

Naturally, the invention is not limited exclusively to the abovementioned embodiments, but can be varied within the scope of the subsequent claims.

## Claims

1. A device for the diffusion of substances between two fluids (A, B) separated by semipermeable membranes (2), which membranes are arranged in pairs for conducting a first fluid (A) including oxygen between the membranes of the pairs and a second fluid (B) such as blood outside the membranes with diffusion of one or more substances from the first fluid (A) to the second fluid (B) through the membranes (2), including spacer elements in the form of plates (1), separating the pairs of membranes from each other, and intended to work as a so-called oxygenator, characterized in that means (7, 8, 63) are arranged for combining said diffusion with a subsequent direct mixing of the first fluid (A) into the second fluid (B) and in that said spacing plates (1) are provided with channels which are adapted so as to conduct the second fluid (B) mixed with bubbles of said first fluid (A) along the outside of the membranes (2) in the said pair of membranes (2, 2).

2. A device in accordance with claim 1, characterized in that at least one membrane (2) in each pair of membranes (2, 2) is provided with apertures (7; 63) at its end downstream in respect of the first fluid (A) for the direct mixing in of the first fluid (A) into the second (B).

3. A device in accordance with claim 1, characterized in that the spacing plates (1) are separated from a third fluid (C) at their side remote from the said pair of membranes (2, 2) by means of further membranes (3), which preferably are also arranged in pairs (3, 3) so as to enclose between them the third fluid (C), intended for the heating or other tempering of the second fluid (B).

4. A device in accordance with claim 3, characterized in that the inlet and outlets of said first and second fluids (A, B) are arranged for a counter-flow.

5, A device in accordance with claim 3, characterized in that the inlets and outlets of said second and third fluids (B, C) are arranged for a counter-flow.

6. A device in accordance with any one of the preceding claims, characterized by means (11) of the eliminating of the excess of the first fluid (A) from the second fluid (B) after the two fluids have been mixed with one another.

7. A device in accordance with any one of the preceding claims with two or more spacing plates (1) connected in parallel for the conducting of the said second fluid (B), characterized by means (89, 90, 91) adapted so as to conduct this fluid (B) only to certain ones of the said plates (1) in order to vary the capacity of the device.

8. A device in accordance with claim 7, characterized in that the plates (1) are provided with aligned apertures (40) which form a shaft passing through a stack of spacing plates for the introduction of the second fluid (B), channels (41—47) being arranged in the spacing plates (1) for the conducting of the second fluid from the said shaft (40) to the active part (48) of the spacing plate (1), the said means (89, 90, 91) for conducting fluid only to certain ones of the said plates being constituted of shut-off elements for the said channels.

9. A device in accordance with claim 8, characterized in that the said shut-off elements consists of a tubular plunger (89) penetrating into the shaft to a greater of lesser extent, which with its outer side can shut off one or more of the said channels.

## Patentansprüche

1. Vorrichtung für die Diffusion von Substanzen zwischen zwei Fließmitteln (A, B), welche durch halbdurchlässige Membranen (2) getrennt sind, die in Paaren angeordnet sind, für das Leiten eines ersten Fließmittels (A) mit Sauerstoff zwischen den Membranen des Paares und eines zweiten Fließmittels (B), wie z.B. Blut, außerhalb der Membranen mit Diffusion einer oder mehrerer Substanzen aus dem ersten Fließmittel (A) zu dem zweiten Fließmittel (B) durch die Membranen (2), mit Abstandselementen in der Form von Platten (1), welche die Membranpaare voneinander trennen und dafür vorgesehen sind, als sogenannte Sauerstoffanreicherungseinrichtung zu arbeiten, dadurch gekennzeichnet, daß Einrichtungen (7, 8, 63) angeordnet sind für die Kombination der Diffusion mit einem nachfolgenden direkten Einmischen des erstein Fließmittels (A) in das zweite Fließmittel (B) und daß die Abstandsplatten (1) mit Kanälen versehen sind, die geeignet derart ausgestaltet sind, daß sie das zweite Fließmittel (B), welches mit Blasen der ersten Fließmittels (A) vermischt ist, längs der Außenseite der Membrane (2) in dem Paar von Membranen (2, 2) führen.

2. Vorrichtung nach Anspruch 1, dadurch gekennzeichnet, daß mindestens eine Membran (2) in jedem Paar von Membranen (2, 2) mit Öffnungen (7; 63) an ihrem abstromseitigen Ende bezüglich des ersten Fließmittels (A) versehen ist für das direkte Einmischen des ersten Fließmittels (A) in das zweite (B).

3. Vorrichtung nach Anspruch 1, dadurch gekennzeichnet, daß die Abstandsplatten (1) an ihrer von dem Paar von Membranen (2, 2) entfernten Ende mittels weiterer Membranen (3) von einem drittel Fließmittel (C) getrennt sind, wobei die weiteren Membranen (3) vorzugsweise auch in Paaren (3, 3) so angeordnet sind, daß sie zwischen sich das dritte Fließmittel (C) einschließen, welches für die Erwärmung oder ein anderes Temperieren des zweiten Fließmittels (B) vorgesehen ist.

4. Vorrichtung nach Anspruch 3, dadurch gekennzeichnet, daß die Einlässe und Auslässe des ersten und zweiten Fließmittels (A, B) für eine Gegenströmung angeordnet sind.

5. Vorrichtung nach Anspruch 3, dadurch gekennzeichnet, daß die Einlässe und Auslässe der zweiten und dritten Fließmittel (B, C) für eine Gegenströmung angeordnet sind.

6. Vorrichtung nach einem der vorhergehenden Ansprüche, gekennzeichnet durch Mittel (11) für das Abscheiden des Überschusses des ersten Fließmittels (A) aus dem zweiten Fließmittel (B), nachdem die zwei Fließmittel miteinander vermischt worden sind.

7. Vorrichtung nach einem der vorhergehenden Ansprüche mit zwei oder mehreren Abstandsplatten (1), die parallel verbunden sind für das Leiten des zweiten Fließmittels (B), gekennzeichnet durch Mittel (89, 90, 91), die geeignet derart ausgestaltet sind, daß sie dieses Fließmittel (B) nur zu gewissen Platten (1) leiten, um die Kapazität der Vorrichtung zu verändern.

8. Vorrichtung nach Anspruch 7, dadurch gekennzeichnet, daß die Platten (1) mit in Flucht liegenden Öffnungen (40) versehen sind, die einen Durchlaß oder Schaft bilden, welcher durch einen Stapel von Abstandsplatten für das Einführen des zweiten Fließmittels (B) hindurchgeht, und daß Kanäle (41—47) in den Abstandsplatten (1) angeordnet sind für das Herausleiten des zweiten Fließmittels aus dem Schacht (40) zu dem aktiven Teil (48) der Abstandsplatte (1), wobei die Einrichtungen (89, 90, 91) für das Leiten von Fließmittel nur zu einigen Platten aus Abschaltelementen für die Kanäle bestehen.

9. Vorrichtung nach Anspruch 8, dadurch gekennzeichnet, daß die Abschaltelemente aus einem rohrförmigen Kolben (89) bestehen, welcher in den Schacht mehr oder weniger eindringt, wobei seine Außenseite einen oder mehrere Kanäle abschalten kann.

**Revendications**

1. Un dispositif pour la diffusion de substances entre deux fluids (A, B) séparés par des membranes semiperméables (2), membranes qui sont agencées par paires pour conduire un premier fluide (A) contenant de l'oxygène entre les membranes des paires et un second fluide (B), tel que du sang, à l'extérieur des membranes, avec diffusion d'une ou plusieurs substances à partir du premier fluide (A) vers le second fluide (B), à travers les membranes (2), comprenant des éléments intercalaires sous la forme de plaques (1), qui séparent les paires de membranes les unes des autres, et destiné à être utilisé en tant que dispositif appelé oxygénateur, caractérisé en ce que des moyens (7, 8, 63) sont agencés de manière à combiner ladite diffusion avec un mélange direct ultérieur du premier fluide (A) dans le second fluide (B) et en ce que lesdites plaques intercalaires (1) sont munies de canaux qui sont conçus pour conduire le second fluide (B) mélangé avec des bulles dudit premier fluide (A) le long de l'extérieur des membranes (2) dans lesdites paires de membranes (2, 2).

2. Un dispositif suivant la revendication 1, caractérisé en ce qu'au moins une membrane (2) de chaque paire de membranes (2, 2) est munie d'ouvertures (7; 63) à son extrémité aval par rapport au premier fluide (A), pour le mélange direct du premier fluide (A) dans le second (B).

3. Un dispositif suivant la revendication 1, caractérisé en ce que les plaques intercalaires (1) sont séparées d'un troisième fluide (C) au niveau de leur côté éloigné de ladite paire de membranes (2, 2), au moyen d'autres membranes (3), qui de préférence sont également agencées par paires (3, 3) de façon à enfermer entre elles le troisième fluide (C), destiné à chauffer ou à tempérer autrement le second fluide (B).

4. Un dispositif suivant la revendication 3, caractérisé en ce que les entrées et les sorties desdits premier et second fluides (A, B) sont agencées pour obtenir un contre-courant.

5. Un dispositif suivant la revendication 3, caractérisé en ce que les entrées et les sorties desdits second et troisième fluides (B, C) sont agencées pour obtenir un contre-courant.

6. Un dispositif suivant l'une quelconque des revendications précédentes, caractérisé par des moyens (11) pour éliminer l'excès du premier fluide (A) du second fluide (B) après que les deux fluides aient été mélangés l'un avec l'autre.

7. Un dispositif suivant l'une quelconque des revendications précédentes, comportant deux plaques intercalaires (1) ou plus montées en parallèle pour conduire ledit second fluide (B), caractérisé par des moyens (89, 90, 91) conçus de manière à conduire ce fluide (B) uniquement vers certaines desdites plaques (1) afin de faire varier la capacité du dispositif.

8. Un dispositif suivant la revendication 7, caractérisé en ce que les plaques (1) sont munies d'ouvertures appropriées (40) qui forment un axe qui traverse un empilement de plaques intercalaires pour l'introduction du second fluide (B), des canaux (41—47) étant agencés dans les plaques intercalaires (1) pour conduire le second fluide dudit axe (40) vers la partie active (48) de la plaque intercalaire (1), lesdits moyens (89, 90, 91) pour conduire du fluide uniquement ver certaines desdites plaques étant constitués par des éléments de fermeture pour lesdits canaux.

9. Un dispositif suivant la revendication 8, caractérisé en ce que lesdits éléments de fermeture sont constitués par un plongeur tubulaire (89), pénétrant plus ou moins dans l'axe, dont la face extérieure peut fermer un ou plusieurs desdits canaux.

# Fig.1

Fig.2

Fig.3

Fig.4

Fig. 7

Fig. 6

Fig. 5

Fig. 8

Fig. 9

## Fig. 10

51 49 56 55 59 1a
49 51 54 57 58 60

## Fig. 12

1a 51 49 50
60 49

## Fig. 11

65 61 1a
60 66

## Fig. 13

60 44 43 69 67 1a
70 42 41 40 68 64

Fig. 14

0 003 495

6

## Fig.15

## Fig. 16

## Fig. 17

62′

61′

57′

2a

2a

63

40′

64′

*Fig. 18*

*Fig. 19*

*Fig. 20*

*Fig. 21*